# EUROPEAN PATENT APPLICATION

(11) **EP 2 348 479 A1**
(43) Date of publication of application: **27.07.2011**
(21) Application number: 10252076.4
(22) Date of filing: 08.12.2010
(51) Int. Cl.: G06Q 50/00, G06F 19/00

(54) **Motivational profiling for behavioral change technologies: a state-trait approach**

(30) Priority: 23.12.2009 US 645904
(71) Applicant: Intel Corporation, Santa Clara, CA 95052 (US)
(72) Inventor: Morris, Margret E., Portland, OR 97209 (US); Whitlinger, David L., New York, NY 10023 (US); Philipose Matti, Seattle, WA 98115 (US)
(74) Representative: Beresford, Keith Denis Lewis

(57) **Abstract**

An electronic system for health based behavior modification may receive real time data to assess behavioral state changes. The real time data may be used as an input to a database that matches states and trait-based typologies to determine motivational profiles. A rules database may be used to develop recommended motivational coaching changes based on the real time inputs.

## Description

### Background

This relates generally to an electronic device for facilitating behavior modification. Behavior modification is required to improve physical and emotional health, prevent disease and manage existing conditions. Changes in behavior are often also required to improve life satisfaction, appearance, professional success and the quality of relationships. Behavior change is difficult to start and difficult to maintain, as evidenced by the souring rates of obesity and other "lifestyle disorders" and endless best seller books for weight loss and self improvement.

### Brief Description of the Drawings

Figure 1 is a chart of trait-based types matrixed with states according to one embodiment;
Figure 2 is a schematic depiction of one embodiment of the present invention; and
Figure 3 is a flow chart for one embodiment of the present invention.

### Detailed Description

Behavior modification is required to improve physical and emotional health, prevent disease and manage existing conditions. Changes in behavior are often also required to improve life satisfaction, appearance, professional success and the quality of relationships. Behavior change is difficult to start and difficult to maintain, as evidenced by the souring rates of obesity and other "lifestyle disorders" and endless best seller books for weight loss and self improvement.

Many factors are at play in behavioral change, but one, the motivation to change, may be facilitated by the present invention.

Behavior modification may be dependent on certain traits in certain states. A trait is a characteristic that remains generally unchanged over time. A state is a characteristic that changes over time. Generally, traits can be distinguished from states based on the rate of change, with traits being characteristics that change more slowly than states change.

A behavior modification may be successful until a state change occurs. Thus, an effective behavior modification technique responds to state changes. According to some embodiments of the present invention, by accepting real time inputs, state changes can be detected, behavior modification techniques can be modified instantaneously in view of state changes, and more effective behavior modification may be the result in some embodiments.

Trait-based typologies are combinations of traits that influence health status or health related behavior. Examples of traits include ethnicity, sex, family medical history, personality, health beliefs, professional autonomy, self-efficacy, intelligence, life-style, habits, outlook, socioeconomic status, and educational level.

States are more dynamic characteristics that influence health behavior. Examples of states are stress, loneliness, progress towards health goals, recent lapses from exercise targets, mood, recent behaviors, and physical health metrics, such as weight, blood pressure, triglyceride level, pulse rate, and health engagement.

Motivational profiles describe what drives or influences health behaviors at different moments for a given type of person having a given set of trait-based typologies. These motivational profiles matrix trait-based types and states. Thus, the result is a model in the form of a matrix or table referenced by a computer program. The motivational profile offers guidelines for self-reflection and health coaching. Examples of motivational profiles include appearance, belongingness, and competitiveness.

Profiles describe what motivational strategies are most likely to be effective at different points in time for a given type of person, given the person's current state. Thus, for example, as a man in midlife progresses on an exercise plan, the desire to extend life time and health function "to be available for the children" might be the motivation for behavior modification. As time went on, his wife's appreciation of his weight loss and improved appearance motivates him to continue a difficult life style change, whose motivation had begun to dip slightly. Eventually, his routine of running with friends develops into a desire to compete. At that point, an upcoming 10k race motivated him to continue running. For this person, the state shifts that triggered an evolution in motivational profile related to his health behaviors and to his relationships with his wife and peers. For others, with a different trait-type, the state shifts that trigger evolution as a motivational profile could be mood changes, stressful circumstances, or other types of life events.

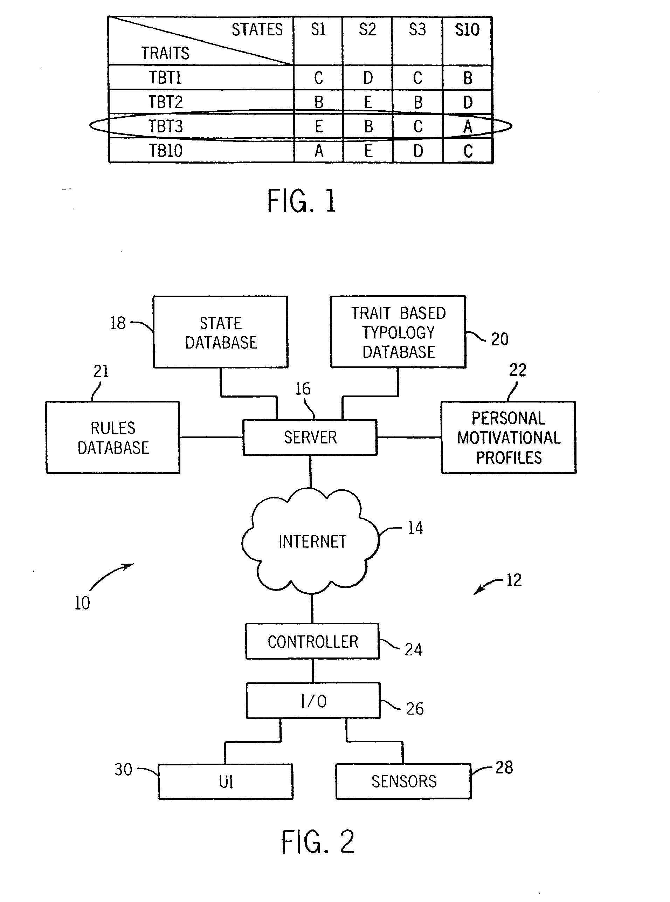

Thus, referring to Figure 1, a chart, shown in written form, may be implemented in software as a table. The rows of the chart may correspond to trait-based types, such as trait-based type (TBT1), trait-based type (TBT2), etc. The rows may be a large number of trait-based types that have been identified in psychological research in one embodiment. Individual persons may be matched to their trait-based types using interviews or machine generated questionnaires, to mention two examples. Each of the columns corresponds to a different state.

The states may be refined for specific applications, such as exercise, and even a more specific type of exercise, such as cycling. In software embodiments, software modules may be offered to implement different more focused or specialized types of behavior modification. Thus, a competitive cyclist may use one module while someone trying to lose weight might use another module, both for cycling. Different modules may be available for other functions, such as running, swimming, smoking cessation, reducing drug dependency, and the like.

As another example of how changing states may affect motivational profile, a teenage girl may be initially motivated to exercise to improve her appearance. Over time, she may join a tennis club that provides a sense of companionship and provides peer support. But eventually she may start competing. These motivational profiles evolve from appearance to companionship and then to competition, with shifts in states. In this example, the state shift is progress towards health goals. As she twh progresses, she wants to affiliate and then compete. An electronic system can address these general motivational themes so that individuals can better motivate themselves and coaching can preempt drop offs at different junctures.

The following example is merely to illustrate the operation of the chart shown in Figure 1. A middle-aged recently divorced man may be assigned to trait-based type (TBT) 3, circled in Figure 1. A state S1 may be initially assigned based on questions generated either by a coach or by the machine. The response to these questions results in the person being identified initially as trait-based type 3. His initial state of S1 was assigned because he was motivated by a desire to extend his lifespan in order to Lake care of his kids.

Over time, another state S2 (Figure 1) may be identified. In this example, the states may be macro-states or states which exhibit large changes from a previous state. For example, state S2 may arise because the man is now motivated by a sense of belonging. As an example, state information may be developed that suggests that the person is no longer motivated by lifespan enhancement, but responses to questions revealed that he might be responsive to increased social interaction.

Another example of a macro-state, for state S3 in Figure 1, the motivational profile may be to suggest becoming more competitive. It may arise because the man is no longer motivated by a desire to belong, perhaps because he has lost weight and has become more fit. Over time, the man may move to state S10 in which the motivational profile is about the drive to be attractive. Between these macro-states may be an enormous number of micro (??)states in which smaller changes have occurred and are detected so that the recommendations transform from one motivational profile to another as the person transforms from a situation more attuned to one type of motivational profile than the other. Thus, macro-changes may be responded to by the computer, as well as micro-changes, on a daily or hourly basis. The micro-changes can be as small as, during a given exercise session, identifying data which suggests a state change.

Another example of a microstate is seen in a woman who used a stationary cycling bicycle who has done the same exercise a number of days in a row, but at the same time, under the same exercise, is putting out much less effort. This could precipitate a question on the computer display about why she is working less hard, implicit physiological measures related to exertion and ultimately a presentation of different motivational stimuli. If the woman expressed fatigue but showed a heart rate, other queries could help determine a more accurate cause for her low performance. For example, it could determine if but was losing motivation because of changes in mood, self-esteem, professional pressures etc. The system may be organized by trait-based types, maintaining a list of possible states a person of a certain trait-based type may be in, a list of actions that can be performed to increase motivations, and the probability that a given action can move the individual from one level of a health behavior to another. Given a goal level that the person wants to reach, such as jogging five miles, the system computes a policy, which is a table matching each state to the optimal action plan to be taken in that state in order to achieve the goal.

One approach may be policy iteration or iterating over the space of all the possible policies until the optimal policy is found. Policy iteration is exponential in the number of states of a person and may involve the use of heuristics for better performance.

Initially, a best guess or starting point is determined based on psychological research and then refined based on the person's behavior and usage of the system. The initial policy may be developed by identifying the trait-based type based on questionnaire responses and referencing the row of the trait/state matrix to determine how each state maps onto a motivational profile.

Moreover, the computer can also learn adaptively as time passes. While the computer or professional may make an initial assessment of trait-based type, based on the person's behavior, including what action the user takes in response to advice provided by the machine, the person's trait-based type can be continually reconsidered. Thus, a person who was initially judged to be trait-based type 3, at some point may be recategorized to trait-based type 2 and it may be learned by the machine that the advice given with this trait-based type assessment tends to be more effective. Such responses would confirm that judgment of the trait-based type.

The motivational profile for a person in a given state directly determines what motivating action should be taken. These actions are messages that frame health goals in terms of the profile. For example, the person might be advised by a graphical user interface that "this might be a good time to think about combining exercise with socializing." The entries in each row in Figure 1 map states to action. On the basis of the initial guess for an optimal policy for a given person, and the person's actual responses to motivational prompts, as well as responses from biological metric sensors, a local search is run around the seed policy to learn or develop an optimal policy, customized to the person's actual behavior. Thus, in some embodiments, the system may use experience sampling, also called ecological momentary sampling of mood and behaviors, sensing physiological parameters related to exercise and stress patterns, periodic questionnaires to assess life styles and physical health indicators, and initial administration of questionnaires to assess personality, life style, family history, and individual health history.

Multiple touch points, such as personal computers, mobile devices, or wearable sensors may be used to gather data for the initial typology and dynamic calibration of motivational profiles. The profiling tool recalibrates frequently in response to these data streams.

Thus, according to one embodiment, shown in Figure 2, the system 10 may be implemented over the Internet 14. In another embodiment, a network, such as a wireless network, may be used instead. A website or server 16 may be connected to the Internet. The server 16 analyzes data and provides adjusted responses. For example, the server 16 may be coupled to a rules database 21, a state database 18 that records the possible states, a trait-based typology database 20 which records the possible trait-based typologies, and a personal motivational profiles database 22, developed by the server 16, based on information about states, trait-based typologies, and the application of rules.

Information may be provided to the server to develop the personal motivational profiles stored in the database 22 through a user device 12 including user interface (UI) 30, coupled to an input/output (I/O) adapter 26 and a controller 24. The controller 24 may be a processor in one embodiment. The input/output adapter 26 also may be coupled to biological metric sensors 28. These sensors may develop information such as weight, heart rate, blood pressure, blood characteristics, user habits or actions, personal appearance recorded via video camera and analyzed automatically to develop appearance information, sounds to detect voice patterns, and moisture to detect nervousness or sweating, to mention a few examples. In addition, the sensors may be coupled to sport or exercise equipment to provide information on heart rate, calories, speed (e.g. cycling computer), distance, and exercise performance.

The user device 12 may be any of a variety of personal computing devices that may be standalone or may be user wearable. Thus, examples of devices 12 include exercise equipment with computers within them, scales, wearable computers, home personal or laptop computers, set top boxes, wristwatch computers, and cell phones. In many cases, the devices 12 may be coupled to the Internet 14 or another network through a wireless connection. In one embodiment, in which the device 12 is a cell phone, the connection may be over a cellular telephone network. For example, the cellular telephone may run an application that collects information about current physiological and behavioral states. It could, for example, collect information in response to questions, detect rate of athletic performance in cycling, walking, running, or the like, determine the distance traveled during exercise, and the speed at which the exercise was performed, to mention a few examples. In many cases, a variety of devices 12 may all link together over a network to a base station that then uploads the information to the server 16 over the Internet 14, as an example.

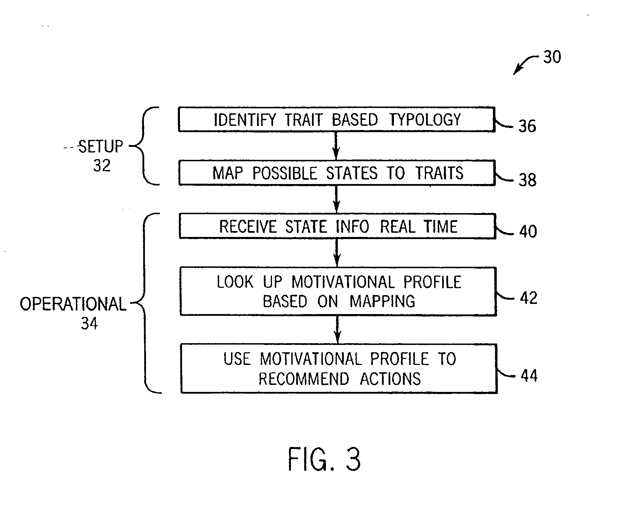

Referring to Figure 3, in some embodiments, the sequence 30 may be implemented in software. The software may include a set of instructions that may be stored in the rules database 21 for execution by the server 16, in one embodiment. Thus, a computer readable medium, such as a semiconductor, optical, or magnetic storage may store the sequence of instructions for execution by a processor or controller.

The sequence 30, in one embodiment, may include two different modes. The first mode 32 is a set up mode wherein data is initially collected. The second mode 34 is an operational mode where an initial profile is adjusted based on real time data inputs, including those from sensors and questionnaires provided through the user interface 30, through the input/output adapter 26, to the controller 24, and then forwarded over the Internet 14 to the server 16, in one embodiment.

In the set up mode, a trait-based type is identified, normally based on responses to a machine generated questionnaire, as indicated in block 36. Then, the possible states are mapped to the trait-based types in block 38 to develop a matrix of personal motivational profiles stored in the database 22, as indicated in block 38. More particularly, the personal motivational profiles 22 may be the result of mapping states in the state database 18 with trait-based typologies in the database 20. Potential actions based on those sets of matched states and trait-based typologies may be developed based on the rules database 21 and stored in connection with a given user and a personal motivational profile specific to a particular user but stored in the database 22.

During the operational mode, state-based information is received in real time (block 40) and this may include information determined passively through sensors 28 with or without the user's awareness. Thus, while the user is exercising, the user may be connected to a variety of sensors which may collect information without requiring any action by the user. In addition, the user may provide information over a user interface 30 in response to prompts or as desired by the user. Next, in block 42, the motivational profile is looked up based on the mapping achieved in block 38, given new information about the state determined in block 40. Then the motivational profile is used to recommend action, as indicated in block 44.

In addition to performance-based metrics, which may be derived by sensors, regular journal entries may be requested. To create the journal entries, the user may be asked to respond to questions on a daily or other basis from which state changes can be detected. But the state change may also be detected by performance based sensors that sense that for the same exercise, the same level of performance is no longer being achieved. This would precipitate more questions that suggest that a change has occurred from the state in which the motivational profile of extending life is no longer valid and a new motivational profile must be generated in order to maintain interest. Thus, in response to state changes, the new motivational profile may be creating a sense of belongingness. As a result, in response to state information, the user may be given prompts, suggestions, or advice to do things that enhance a sense of belonging. Examples could be "Have you considered contacting a friend to exercise with you?" or "Can you imagine joining an exercise club? Or the image and contact information of a friend with might appear on the screen of the individual's phone.

References throughout this specification to "one embodiment" or "an embodiment" mean that a particular feature, structure, or characteristic described in connection with the embodiment is included in at least one implementation encompassed within the present invention. Thus, appearances of the phrase "one embodiment" or "in an embodiment" are not necessarily referring to the same embodiment. Furthermore, the particular features, structures, or characteristics may be instituted in other suitable forms other than the particular embodiment illustrated and all such forms may be encompassed within the claims of the present application.

While the present invention has been described with respect to a limited number of embodiments, those skilled in the art will appreciate numerous modifications and variations therefrom. It is intended that the appended claims cover all such modifications and variations as fall within the true spirit and scope of this present invention.

## Claims

1. A method comprising:
electronically collecting state based information relevant to motivation in real time;
electronically identifying a motivational profile based on an electronically stored mapping of possible motivational-based states mapped to trait-based typologies; and
recommending an action through a user interface of an electronic device using said motivational profile.

2. The method of claim 1 including developing a table that maps states to trait-based typologies.

3. The method of claim 1 including providing an initial policy for recommended motivational action and modifying that policy based on real time information received thereafter.

4. The method of claim 1 including collecting information about real time states and forwarding said information to a server over the Internet.

5. The method of claim 1 including collecting real time biological state information including at least one of the following of blood pressure, pulse rate, weight, speed, distance or performance.

6. The method of claim 1 including receiving electronic questionnaire responses to identify trait-based typologies.

7. The method of claim 1 including electronically providing motivational coaching for behavior modification that changes based on the user's current motivational state.

8. The method of claim 7 including enabling an electronic device to match states with trait-based typologies and to determine motivational profiles and thereby to select one of a plurality of recommended behavioral modification coaching techniques and forwarding the selected technique to the user over an input/output interface.

9. An apparatus comprising:
a controller;
a biometric sensor coupled to said controller; and
a storage coupled to said controller storing software to electronically collect state based information from said sensor in real time relevant to motivation and to recommend a course of action by mapping a trait-based typology to the user's current motivational state.

10. The apparatus of claim 9 wherein said apparatus is a cell phone.

11. The apparatus of claim 9 wherein said apparatus is an exercise device.

12. The apparatus of claim 9 wherein said storage stores a table that maps trait-based typologies to motivational states.

13. The apparatus of claim 9 wherein said storage stores instructions to pose questions to a user over an electronic interface in order to obtain information about the user's current motivational state.

14. The apparatus of claim 9 wherein said apparatus makes an initial assessment of trait-based typology and, based on subsequent use of the apparatus, reassesses said initial trait-based typology assessment.

15. A computer readable medium storing instructions that are executed by a computer to carry out all of the steps of a method as claimed in any one of claims 1 to 8.
